# EUROPEAN PATENT APPLICATION

(11) **EP 3 243 490 A1**
(43) Date of publication of application: **15.11.2017**
(21) Application number: 16735106.3
(22) Date of filing: 08.01.2016
(51) Int. Cl.: A61F 13/14, A41C 1/08, A41D 13/05, A61F 5/02

(54) **WAIST BELT**

(30) Priority: 08.01.2015 JP 2015002658
(71) Applicant: Daiya Industry Co., Ltd., Okayama-shi, Okayama 701-0203 (JP)
(72) Inventor: MATSUO, Masao, Okayama-shi Okayama 701-0203 (JP); KAWAKAMI, Masayuki, Okayama-shi Okayama 701-0203 (JP); UCHIDA, Takahiro, Okayama-shi Okayama 701-0203 (JP)
(74) Representative: de Arpe Fernandez, Manuel
(86) International application number: PCT/JP2016/050567
(87) International publication number: WO 2016/111367

(57) **Abstract**

Provided is a waist belt (1) including: a left inner tightening belt (43) extended from a left main belt (2) and folded back through a left folding-back ring (33) of a right main belt (3); a right inner tightening belt (53) extended from the right main belt (3) and folded back through a right folding-back ring (23) of the left main belt (2); a left subsidiary tightening belt (6) extended leftward from the right main belt (3); a right subsidiary tightening belt (7) extended rightward from the left main belt (2); a left outer tightening belt (44) connected to the left subsidiary tightening belt (6), folded back through a left fixation ring (45) folded back through a left linking ring (433) of the left inner tightening belt (43), and connected to the left main belt (2), the left fixation ring (45) being configured to be attached to and detached from the left main belt (2); and a right outer tightening belt (54) connected to the right subsidiary tightening belt (7), folded back through a right fixation ring (55), folded back through a right linking ring (533) of the right inner tightening belt (53), and connected to the right main belt (3), the right fixation ring (55) being configured to be attached to and detached from the right main belt (3). The waist belt 1 absorbs differences in circumferential length of a waist portion, and does not impair the function of squeezing tight around the waist even when a user moves.

## Description

### Field

The present invention relates to a waist belt that squeezes tight around a waist portion of a user by being worn on a wide part of the waist portion.

### Background

A waist belt that squeezes tight around a waist portion of a user by being worn on a wide part of the waist portion has a main belt inevitably having a wide width. The waist belt therefore needs to be creatively designed so as to make full surface contact with the waist portion. For example, a waist belt (a belt device to wear on a waist portion) disclosed in Patent Literature 1 includes a structure having segmental belts extended in two, upper and lower stages leftward and rightward from a waist-portion covering part worn on a wide part of the waist portion. Each of the segmental belts is made of a stretchable material, so that the segmental belts wound around in two, upper and lower stages can bring the waist-portion covering part into full surface contact with a wide part of a waist portion of a user and squeeze tight around the waist portion.

### Citation List

### Patent Literature

Patent Literature 1: Japanese Patent Application Laid-open No. 2002-095686

### Summary

### Technical Problem

As described above, a waist belt that squeezes tight around a waist portion of a user by being worn on a wide part of the waist portion inevitably has a wide width. Unless differences in circumferential length that occur between upper and lower parts of the waist portion are appropriately handled, the waist belt is kept from entirely making uniform surface contact with the waist portion and therefore cannot fully perform the function of squeezing tight around and fixing the waist portion. Particularly if the user is a female, the differences in circumferential length are not negligible. For example, a wider waist belt is more likely to have a problem of having the waist belt apart from a constricted part of the waist portion or having the waist belt squeezing excessively tight around a part of the waist portion other than the constricted part.

The waist belt disclosed in Parent Literature 1 is intended to absorb the above-described differences in circumferential length by having the segmental belts in the two, upper and lower stages wound round an abdominal part and a lower abdominal part, respectively. However, the segmental belts in two, upper and lower stages are joined to a waist portion, and the individual segmental belts are therefore not expected to achieve full surface contact with the waist portion. Each of the segmental belts is made of a stretchy material. In addition, manners in which the individual segmental belts are stretched are independent from one another. Thus, it cannot be said with certainty that the segmental belts in two, upper and lower stages fully function to squeeze tight around and fix a waist portion because the segmental belts become loose and tight in response to moves of a user thereof even when having been set so as to adequately squeeze at the beginning of being worn.

It is considered that the idea of separating a squeezing device into multiple stages in a waist belt having a wide width serves as a useful reference. Regarding the structure of the waist belt disclosed in Patent Literature 1, however, room of examination still remains in terms of actual conditions of the waist belt at the time of being worn. The present invention is aimed at providing a waist belt that squeezes tight around a waist portion of a user by being worn on a wide part of the waist portion, the waist belt being operable to absorb differences in circumferential length in the waist portion, keep the function of squeezing tight around the waist portion unaffected even when the user moves, and keep a main belt making wide surface contact with the waist portion.

### Solution to Problem

A waist belt includes: a nonstretchable left main belt; a nonstretchable right main belt; a nonstretchable left inner tightening belt; a nonstretchable left outer tightening belt; a nonstretchable right inner tightening belt; a nonstretchable right outer tightening belt; a stretchable left subsidiary tightening belt; and a stretchable right subsidiary tightening belt. The left main belt includes a right folding-back ring in the lower half of the right end thereof. The right main belt includes a left folding-back ring in the lower half of the left end thereof. The left inner tightening belt has the right end thereof connected to the lower half of the right end of the left main belt, is passed through the left folding-back ring and folded back leftward, and includes a left linking ring at the left end thereof. The right inner tightening belt has the left end thereof connected to the lower half of the left end of the right main belt, is passed through the right folding-back ring and folded back rightward, and has a right linking ring at the right end thereof. The left subsidiary tightening belt includes an insertion ring through which the right subsidiary tightening belt is passed, has the right end thereof joined to the upper half of the left end of the right main belt, and is extended leftward. The right subsidiary tightening belt has the left end thereof joined to the upper half of the right end of the left main belt, and is passed through the insertion ring and extended rightward. The left outer tightening belt has the right end thereof connected to the left end of the left subsidiary tightening belt, is passed through a left fixation ring and folded back rightward, is passed through the left linking ring and folded back leftward, and has the left end thereof connected to the left main belt, the left fixation ring being attachable to and detachable from the outer surface of the left main belt. The right outer tightening belt has the left end thereof connected to the right end of the right subsidiary tightening belt, is passed through a right fixation ring and folded back leftward, is passed through the right linking ring and folded back rightward, and has the right end thereof connected to the right main belt, the right fixation ring being attachable to and detachable from the outer surface of the right main belt. Each of the left folding-back and all the other "rings" in the present invention is an annular member made of resin or metal and is preferably fully closed but may have a gap that is small enough to keep the corresponding belt from slipping out (the same applies to the following description).

The waist belt of the present invention includes a set of a left inner tightening ring and a left outer tightening ring and a set of a right inner tightening ring and a right outer tightening ring. In the waist belt of the present invention, the lower halves of the left main belt and the right main belt are strongly tightened by use of the set of the left inner tightening ring and the left outer tightening ring and the set of the right inner tightening ring and the right outer tightening ring. Specifically, the left outer tightening belt pulls the left linking ring with the total of loads acting on the left fixation ring and the right end of the left outer tightening belt that is connected to the left main belt. With the total of respective loads acting on the left linking ring and the right end of the left inner tightening belt that is connected to the right end of the left main belt, the left inner tightening belt pulls the left folding-back ring provided at the left end of the right main belt. Likewise, the right outer tightening belt pulls the right linking ring with the total of loads acting on the right fixation ring and the left end of the right outer tightening belt that is connected to the right main belt. With the total of respective loads acting on the right linking ring and the left end of the right inner tightening belt that is connected to the left end of the right main belt, the right inner tightening belt pulls the right folding-back ring provided at the right end of the left main belt. The left fixation ring and the right fixation ring are configured so as to be able to pull a left outer tightening ring and a left subsidiary tightening ring and to pull a right outer tightening ring and a right subsidiary tightening ring, respectively, at the same time.

The waist belt of the present invention may be configured as: a waist belt including: a nonstretchable left main belt; a nonstretchable right main belt; a nonstretchable left tightening belt; a nonstretchable right tightening belt; a stretchable left subsidiary tightening belt; and a stretchable right subsidiary tightening belt. The left main belt includes a right folding-back ring in the lower half of the right end thereof. The right main belt includes a left folding-back ring in the lower half of the left end thereof. The left subsidiary tightening belt includes an insertion ring through which the right subsidiary tightening belt is passed, has the right end thereof joined to the upper half of the left end of the right main belt, and is extended leftward. The right subsidiary tightening belt has the left end thereof joined to the upper half of the right end of the left main belt, and is passed through the insertion ring and extended rightward. The left tightening belt has the right end thereof connected to the lower half of the right end of the left main belt, is passed through the left folding-back ring and folded back leftward, is passed through the left fixation ring and folded back rightward, and has the left end thereof connected to the right end of the left subsidiary tightening belt, the left fixation ring being attachable to and detachable from the outer surface of the left main belt. The right tightening belt has the left end thereof connected to the lower half of the left end of the right main belt, is passed through the right folding-back ring and folded back rightward, is passed through a right fixation ring and folded back leftward, and has the left end thereof connected to the right end of the right subsidiary tightening belt, the right fixation ring being attachable to and detachable from the outer surface of the right main belt.

In the waist belt of the present invention simply including a left tightening ring and a right tightening ring, the lower halves of the left main belt and the right main belt are tightened only by pulling a set of the left tightening ring and a set of the right tightening ring individually. Specifically, with the total of respective loads acting on the left fixation ring and the right end of the left tightening belt that is connected to the right end of the left main belt, the left tightening belt pulls the left folding-back ring provided at the left end of the right main belt. Likewise, with the total of respective loads acting on the right fixation ring and the left end of the right tightening belt that is connected to the left end of the right main belt, the right tightening belt pulls the right folding-back ring provided at the right end of the left main belt. The left fixation ring and the right fixation ring are configured so as to be able to pull the left tightening ring and the left subsidiary tightening ring and to pull the right tightening ring and the right subsidiary tightening ring, respectively, at the same time. The waist belt simply including the left tightening ring and the right tightening ring can be configured without a left linking ring and a right linking ring. As compared with the foregoing waist belt of the present invention that includes a set of the left inner tightening ring and the left outer tightening ring and a set of the right inner tightening ring and the right outer tightening ring, this waist belt simply including the left tightening ring and the right tightening ring can be lightweight and does not have the left linking ring and the right linking ring pressed against the left main belt and the right main belt, respectively.

The terms "left", "right", "up", and "down" as used in the present invention refer to "left", "right", "up", and "down" with respect to a user wearing any one of the waist belts. "Left", "right", "up", and "down" in the back-side development view of the waist belt agree with "left", "right", "up", and "down" in the structure thereof. Likewise, "inner" refers to a side relatively near to the user. In other words, a side making contact with the user is expressed as being "inner". "Outer" refers to a side relatively far from the user. In other words, a side that can be seen from the outside is expressed as being "outer". "Left end" refers to a range including a left end. "Right end" refers to a range including a right end. "Intermediate" refers to a range including a midpoint. The terms "left end", "right end", and "intermediate" do not mean only the ends and the midpoint of the left main belt, the right main belt, the left tightening belt, the left inner tightening belt, the left outer tightening belt, right tightening belt, the right inner tightening belt, the right outer tightening belt, the left subsidiary tightening belt, or the right subsidiary tightening belt in a direction in which the corresponding belt extends. The terms "left half", "right half", "upper half", and "lower half" refer to parts obtained by halving the left main belt, the right main belt, the left tightening belt, the left inner tightening belt, the left outer tightening belt, the right tightening belt, the right inner tightening belt, the right outer tightening belt, the left subsidiary tightening belt, or the right subsidiary tightening belt in a rightward/leftward direction or in an upward/downward direction thereof. A "left half" means the left one of parts obtained by the halving in the rightward/leftward direction. A "right half" means the right one of parts obtained by the halving in the rightward/leftward direction. An "upper half" means the upper one of parts obtained by the halving in the upward/downward direction. A "lower half" means the lower one of parts obtained by the halving in the upward/downward direction.

The waist belt of the present invention includes the following on the outer surfaces of the nonstretchable left main belt and the nonstretchable right main belt: in a lower stage, either the nonstretchable left tightening belt or a set of the nonstretchable left inner tightening belt and the nonstretchable left outer tightening belt, and either the nonstretchable right tightening belt or a set of the nonstretchable right inner tightening belt and the nonstretchable right outer tightening belt; and, in an upper stage, the stretchable left subsidiary tightening belt and the stretchable right subsidiary tightening belt. In the waist belt of the present invention, the right end of the left main belt and the left end of the right main belt, which are set opposed to each other, are worn on the waist portion of the user in such a manner as to face each other across the backbone of the user. In the waist belt of the present invention, the widths (length in the upward/downward direction) of the left main belt and the right main belt are determined so that none of the left tightening belt, the left inner tightening belt, the left outer tightening belt, the right tightening belt, the right inner tightening belt, the right outer tightening belt, the left subsidiary tightening belt, and the right subsidiary tightening belt can make direct contact with the user. The left subsidiary tightening belt, the right subsidiary tightening belt, the left folding-back ring, and the right folding-back ring are arranged between the right end of the left main belt and the left end of the right main belt, which are set opposed to each other. Consequently, each of the left main belt and the right main belt that are worn on the waist portion of the user inevitably has a wide width, the maximum of which is a width obtained by adding up the respective widths of the left subsidiary tightening belt, the left folding-back ring, and the right folding-back ring, excluding the right subsidiary tightening belt that is passed through the insertion ring of the left subsidiary tightening belt. The widths of the left main belt and the right main belt can be reduced by having the levels of the left folding-back ring and the right folding-back ring uniformed in such a manner that the left tightening belt or the left inner tightening belt is inserted through the right folding-back ring or that, to the contrary, the right tightening belt or the right inner tightening belt is inserted through the left folding-back ring.

Consequently, in the waist belt of the present invention, when the left fixation ring and the right fixation ring are fixed onto the respective outer surfaces of the left main belt and the right main belt with the left tightening belt and the right tightening belt, or the left outer tightening belt and the right outer tightening belt, being pulled, the left main belt and the right main belt make wide surface contact with the waist portion in a manner such that either the left tightening belt or a set of the left inner tightening belt and the left outer tightening belt and either the right tightening belt or a set of the right inner tightening belt and the right outer tightening squeeze tight around the lower half of the waist portion while the left subsidiary tightening belt and the right subsidiary tightening belt squeeze tight around an upper part of the constricted waist portion. When the user moves, the left subsidiary tightening belt and the right subsidiary tightening belt elongate and contract, and the left main belt and the right main belt are kept in tight contact with the upper half of the constricted waist portion. At the same time, the elongation and contraction of the left subsidiary tightening belt and the right subsidiary tightening belt absorb the move of the user, so that either the left tightening belt or a set of the left inner tightening belt and the left outer tightening belt and either the right tightening belt or a set of the right inner tightening belt and the right outer tightening belt are allowed to follow the move of the user while being kept tense, whereby the left main belt and the right main belt are kept making surface contact with the waist portion.

The structure such that the right subsidiary tightening belt is passed through the insertion ring provided to the left subsidiary tightening belt works to uniform the tension generated by the stretchable left subsidiary tightening belt and the stretchable right subsidiary tightening belt. This means that the relation therebetween may be reversed. That is, another structure may be employed such that: the insertion ring is provided to the right subsidiary tightening belt instead of the left subsidiary tightening belt; the left subsidiary tightening belt has the right end thereof joined to the upper half of the left end of the right main belt, and is passed through the insertion ring and extended leftward; and the right subsidiary tightening belt has the left end thereof joined to the upper half of the right end of the left main belt and is extended rightward. All that the waist belt of the present invention needs to include are: the left subsidiary tightening belt and the right subsidiary tightening belt arranged in an upper stage; and either the left tightening belt or a set of the left inner tightening belt and the left outer tightening belt and either the right tightening belt or a set of the right inner tightening belt and the right outer tightening belt arranged in a lower stage. The waist belt is basically formed line-symmetrically in the rightward/leftward direction. Therefore, regardless of which of the left subsidiary tightening ring and the right subsidiary tightening ring the insertion ring is provided to, the function of keeping the left main belt and the right main belt making surface contact with the waist portion is not impaired.

Various conventionally publicly known attachment/detachment devices are each usable as a device for having the left main belt and the right main belt wound around and worn on the waist portion of the user. It is preferable that the left main belt and the right main belt be integrally wound around the waist portion of the user with one part of a hook-and-loop fastener and the other part of the hook-and-loop fastener formed so as to be attachable to and detachable from each other, the one part being provided on either or both of the inner surfaces of the left end of the left main belt and the right end of the right main belt, the other part being provided on either or both of the outer surfaces of the left main belt and the right main belt. For example, a male surface (hook surface) of a hook-and-loop fastener may be provided on the inner surface of the right end of the right main belt, and a female surface (loop surface) of the hook-and-loop fastener may be provided on the outer surface of the left end of the left main belt. In such a case, wearing of the left main belt and the right main belt that have been wound around the waist portion of the user from the back side is completed once the male surface on the inner surface of the right end of the right main belt is engaged with the female surface on the outer surface of the left main belt.

The left fixation ring not only serves as a pulling device that pulls the left tightening belt or a set of the left inner tightening belt and the left outer tightening belt in the lower stage, and the left subsidiary tightening belt in the upper stage at the same time, but also serves as a restraining device that restrains either the left tightening belt or the set of the left inner tightening belt and the left outer tightening belt, and the left subsidiary tightening belt while keeping these belts pulled. Likewise, the right fixation ring not only serves as a pulling device that pulls the right tightening belt or a set of the right inner tightening belt and the right outer tightening belt in the lower stage, and the right subsidiary tightening belt in the upper stage at the same time, but also serves as a restraining device that restrains either the right tightening belt or the set of the right inner tightening belt and the right outer tightening belt, and the right subsidiary tightening belt while keeping these belts pulled.

Various conventionally publicly known attachment/detachment devices are each usable as a device for having the above-described left fixation ring and the above-described right fixation ring attached and fixed to the left main belt and the right main belt, respectively. It is preferable that: the left fixation ring be positionally fixed with respect to the outer surface of the left main belt with one part of a hook-and-loop fastener and the other part thereof formed so as to be attachable to and detachable from each other, the one part being provided to the left fixation ring, the other part being provided on the outer surface of the left main belt; and the right fixation ring be positionally fixed with respect to the outer surface of the right main belt with one part of a hook-and-loop fastener and the other part thereof formed so as to be attachable to and detachable from each other, the one part being provided to the right fixation ring, the other part being provided on the outer surface of the right main belt. For example, male surfaces of hook-and-loop fastener may be provided on the left tab and the right tab attached to the left fixation ring and the right fixation ring, respectively, and female surfaces of the hook-and-loop fasteners may be provided on the respective outer surfaces of the left main belt and the right main belt. In such a case, the fixation is completed once the left tab of the left fixation ring, which pulls the left tightening belt or a set of the left inner tightening belt and the left outer tightening belt while pulling the left subsidiary tightening belt, is engaged with the female surface provided on the outer surface of the left main belt, and the right tab of the right fixation ring, which pulls the right tightening belt or a set of the right inner tightening belt and the right outer tightening belt while pulling the right subsidiary tightening belt, is engaged with the female surface provided on the outer surface of the right main belt.

### Advantageous Effects of Invention

According a waist belt of the present invention, the waist belt can be worn on a wide part of a waist portion of a user by having differences in circumferential length of the waist portion absorbed, the function of squeezing tight around the waist portion can be left unaffected when the user moves, and a main belt can be kept making wide surface contact with the waist portion. These effects are obtained by pressing the nonstretchable left main belt and the nonstretchable right main belt against the waist portion of the user, the left main belt and the right main belt being to be wound around the waist portion, the pressing being achieved by the combined use of: squeezing tight with either the nonstretchable left tightening belt or a set of the nonstretchable left inner tightening belt and the nonstretchable left outer tightening belt and either the nonstretchable right tightening belt or a set of the nonstretchable right inner tightening belt and the nonstretchable right outer tightening belt; and squeezing tight with the stretchable left subsidiary tightening belt and the stretchable right subsidiary tightening belt.

Attachment and detachment of the left main belt and the right main belt to and from each other using the hook-and-loop fastener facilitates the work of wearing the waist belt of the present invention by winding it around the waist portion of the user. Likewise, fixation of the left fixation ring to the left main belt and fixation of the right fixation ring to the right main belt using the hook-and-loop fasteners enables concurrent tightening of either the left tightening belt or a set of the left inner tightening belt and the left outer tightening belt, either the right tightening belt or a set of the right inner tightening belt and the right outer tightening belt, the left subsidiary tightening belt, and the right subsidiary tightening belt. Tightening either the left tightening belt or a set of the left inner tightening belt and the left outer tightening belt, either the right tightening belt or a set of the right inner tightening belt and the right outer tightening belt, the left subsidiary tightening belt, and the right subsidiary tightening belt is the most laborious work. Facilitating this tightening work therefore reduces the strain on the user.

### Brief Description of Drawings

Fig. 1 is a back-side development view of a waist belt given as an example of the present invention.
Fig. 2 is a front-side development view of the waist belt given as the example.
Fig. 3 is a back-side development view equivalent to Fig. 1 of a waist belt given as a modification example 1.
Fig. 4 is a back-side development view equivalent to Fig. 1 of a waist belt given as a modification example 2.
Fig. 5 is a back-side development view equivalent to Fig. 1 of a waist belt given as a modification example 3.
Fig. 6 is a front-side perspective view of the waist belt given as the example.
Fig. 7 is a back-side perspective view of the waist belt given as the example.

### Description of Embodiments

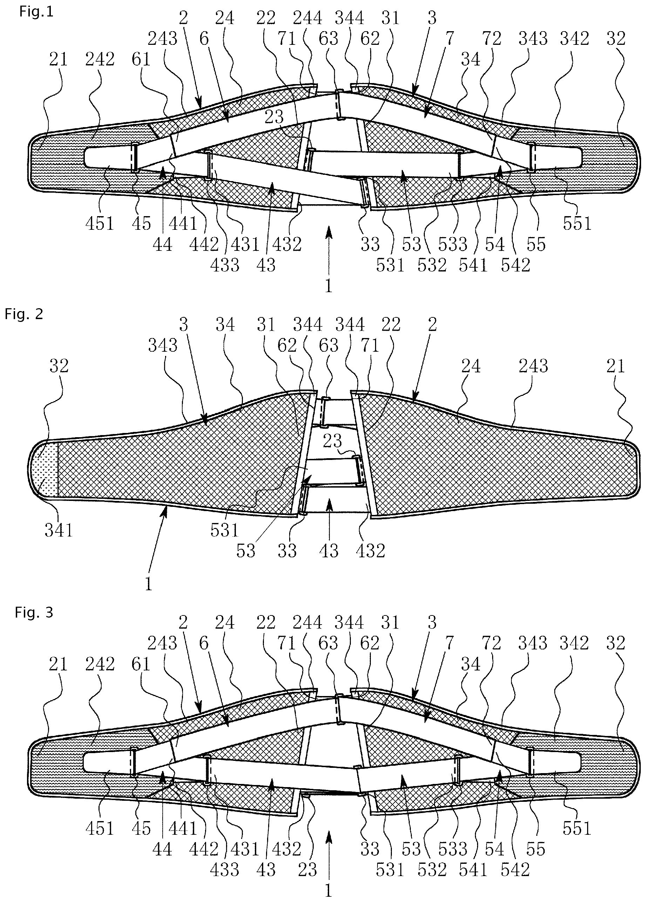

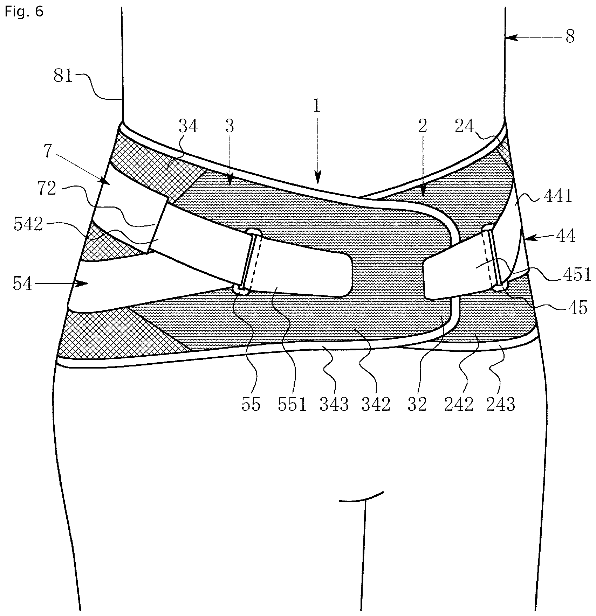

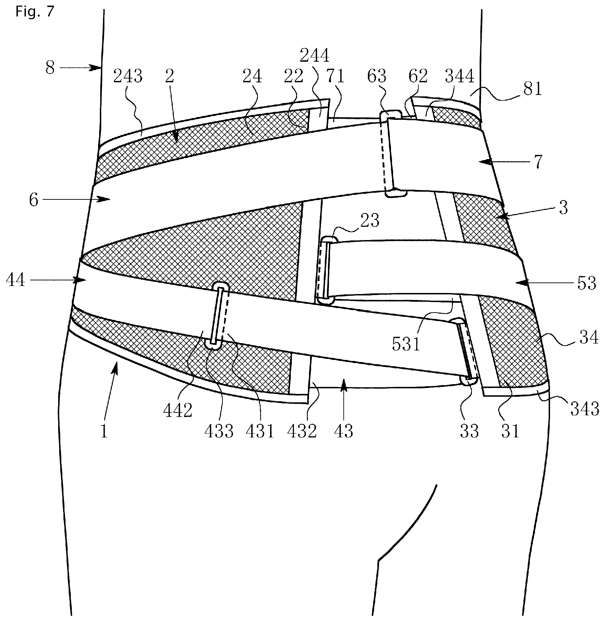

The following describes an embodiment for carrying out the present invention with reference to the drawings. As illustrated in Fig. 1 and Fig. 2 for example, a waist belt 1 of the present invention can be formed substantially in a diamond outer shape viewed from the front side, in which: the neighborhood of the center of the back side thereof to be worn on a waist portion 81 of a user 8 is the widest; and a left main belt 2 and a right main belt 3 extending leftward and rightward narrow toward a left end 21 and a right end 32, respectively. It is thought possible that the left main belt 2 and the right main belt 3 may be formed to have the same widths from the back side to the front side. However, because the user 8 is compressed by immobilization of an abdominal portion thereof, the waist belt 1 preferably has the outer shape of this example that is relatively narrower in the front side when being worn (refer to Fig. 6 and Fig. 7 to be explained later).

The waist belt 1 of this example includes: a left main belt 2, a right main belt 3, a left inner tightening belt 43, a left outer tightening belt 44, a right inner tightening belt 53, and a right outer tightening belt 54 that are nonstretchable; and a left subsidiary tightening belt 6 and a right subsidiary tightening belt 7 that are stretchable. The left main belt 2, the right main belt 3, the left inner tightening belt 43, the left outer tightening belt 44, the right inner tightening belt 53, and the right outer tightening belt 54 are preferably formed of nonstretchable cloth, which does not stretch upward, downward, rightward, or leftward. The left main belt 2 and the right main belt 3 may be entirely flexible so as to fit tightly on the waist portion 81 of the user 8, or may be stretchable upward and downward. Directions in which the left subsidiary tightening belt 6 and the right subsidiary tightening belt 7 stretch are preferably limited to directions of their extension so as to be effectively stretchable in the directions of their extension.

The left main belt 2 and the right main belt 3 have bilaterally symmetric structures. Therefore, descriptions of the right main belt 3 are added in parentheses to those of the left main belt 2, and differences therebetween are described separately. The left main belt 2 (right main belt 3) has, in a development view (Fig. 1) from the back side, a structure such that: at the right end 22 (left end 31) of a belt main body 24 (belt main body 34) made of mesh cloth that tapers from the right end 22 (left end 31) toward the left end 21 (right end 32), an antislip folded-binding part 244 (antislip folded-binding part 344) is provided; an elastic reinforcement resin core is inserted into the antislip folded-binding part 244 (antislip folded-binding part 344); on the outer surface of the left-half area of the belt main body 24 (belt main body 34) from the left end 21 (right end 32), a male surface 242 of a hook-and-loop fastener (male surface 342 of the hook-and-loop fastener) are attached; and binding 243 (binding 343) is provided on the outer rim of the belt main body 24 (belt main body 34) excluding the reinforcement part 244 (reinforcement part 344).

The belt main body 24 (belt main body 34) made of mesh cloth functions not only to allow secure ventilation so as to prevent stuffiness due to the waist belt 1 covering the waist portion 81 of the user 8 but also to reduce the weight of the waist belt 1 to no small extent. The antislip folded-binding part 244 (antislip folded-binding part 344) has an antislip device provided on the front side thereof that makes surface contact with the waist portion 81 of the user 8 and causes the right end 22 (left end 31) of the belt main body 24 (belt main body 34) to follow the waist portion 81 of the user 8 with elastic deformation of the reinforcement resin core inserted therethrough. At the same time, by the action of the reinforcement resin core, the antislip folded-binding part 244 (antislip folded-binding part 344) suppresses or prevents twisting and deformation of the right end 22 (left end 31) due to tensile force generated when being pulled by the left inner tightening belt 43 (right inner tightening belt 53) and the left subsidiary tightening belt 6 (right subsidiary tightening belt 7) that are attached to the right end 22 (left end 31).

In the left main belt 2 (right main belt 3), sewing attachment of the antislip folded-binding part 244 (antislip folded-binding part 344) is utilized to have the right end 432 (left end 531) of the left inner tightening belt 43 (right inner tightening belt 53) connected to the belt main body 24 (belt main body 34) in a lower stage (an upper stage) of the lower half of the right end 22 (left end 31). Therein, the right end 442 (left end 541) of the left outer tightening belt 44 (right outer tightening belt 54) is slipped under a female surface 242 of the hook-and-loop fastener (female surface 342 of the hook-and-loop fastener), thereby being connected to the belt main body 24 (belt main body 34). Furthermore, in the left main belt 2 (right main belt 3), sewing attachment of the antislip folded-binding part 244 (antislip folded-binding part 344) is utilized to have a right folding-back ring 23 (left folding-back ring 33), through which the right inner tightening belt 53 (left inner tightening belt 43) is passed, provided to an upper stage (a lower stage) of the lower half of the right end 22 (left end 31) via an attachment flap attached to the right inner tightening belt 53 (left inner tightening belt 43). The right folding-back ring 23 (left folding-back ring 33) is preferably made of resin.

The right main belt 3 of this example, the male surface 341 of the hook-and-loop fastener is provided on the inner side of the right end 32, as illustrated in Fig. 2. This structure enables the waist belt 1 of this example to be worn in the following manner. With the right end 22 and the left end 31 of the left main belt 2 and the right main belt 3 placed on the waist portion 81 of the user 8 while being opposed to each other, the right main belt 3 wound around from the right side in the same manner as the left main belt 2 is overlaid on the left main belt 2 wound around from the left side and brought into surface contact with an abdominal portion of the user 8, and the male surface 341 of the hook-and-loop fastener provided on the inner surface of the right end 32 of the right main belt 3 is engaged with the female surface 242 of the hook-and-loop fastener provided on the outer surface of the left main belt 2 (refer to Fig. 6 and Fig. 7 to be explained later).

The left inner tightening belt 43 (right inner tightening belt 53) is extended rightward (leftward) from the right end 22 (left end 31), of the left main belt 2 (right main belt 3), to which the right end 432 (left end 531) thereof is connected and passed through the left folding-back ring 33 (right folding-back ring 23) to be folded back leftward (rightward), and has a left linking ring 433 (right linking ring 533) provided at the left end 431 (right end 532). The left inner tightening belt 43 (right inner tightening belt 53) is made of the same nonstretchable belt material as a conventional waist belt or the like of the same kind is made of. The left linking ring 433 (right linking ring 533) is preferably made of resin.

The left outer tightening belt 44 (right outer tightening belt 54) is extended rightward (leftward) from an end edge of the female surface 242 of the hook-and-loop fastener (the female surface 342 of the hook-and-loop fastener) to which the right end 442 (the left end 541) is connected, is passed through the left linking ring 433 (right linking ring 533) and folded back leftward (rightward), is passed further through the left fixation ring 45 (right fixation ring 55) and folded back rightward (leftward), and has the left end 441 (right bed 542) connected to the left end 61 (right end 72) of the left subsidiary tightening belt 6 (right subsidiary belt 7). The left fixation ring 45 (right fixation ring 55) is preferably made of resin. A left tab 451 (right tab 551) provided with a male surface of the hook-and-loop fastener is attached to the left fixation ring 45 (right fixation ring 55). The left outer tightening belt 44 (right outer tightening belt 54) and the left tab 451 (right tab 551) are made of the same belt material as the left inner tightening belt 43 (right inner tightening belt 53).

The left subsidiary tightening belt 6 of this example is a rubber belt stretchable in the direction of extension thereof, has the right end 62 thereof connected to the upper half of the right belt main body 34 by utilizing the sewing attachment of the antislip folded-binding part 344, is connected to an insertion ring 63 at one point, has a part thereof that continues from this point extended leftward from the insertion ring 63, and has the left end 61 thereof connected to the left end 441 of the left outer tightening belt 44. The insertion ring 63 serves as an undeformable through-hole formed in the middle of the left subsidiary tightening belt 6. The right subsidiary tightening belt 7 of this example is a rubber belt stretchable in the direction of extension thereof likewise, has the left end 71 thereof connected to the upper half of the left belt main body 24 by utilizing the sewing attachment of the antislip folded-binding part 244, is passed through the insertion ring 63 and extended rightward, and has the right end 72 thereof connected to the right end 542 of the right outer tightening belt 54.

The waist belt 1 of this example (refer to Fig. 1 and Fig. 2) has, between the right end 22 of the left main belt 2 and the left end 31 of the right main belt 3, the left inner tightening belt 43, the right inner tightening belt 53, the left subsidiary tightening belt 6, and the right subsidiary tightening belt 7 arranged next to each other in this order from the lower side to the upper side, so that the width of the waist belt 1 has a wide width (length in the upward/downward direction) obtained by adding the widths of these belts. Here, if it is desired that a width of a surface making contact with the waist portion 81 of the user 8 be somewhat narrower, the width of the waist belt 1 can be reduced, for example, in the following manner as in the case of a modification example 1 illustrated in Fig. 3. The right inner tightening belt 53 passed through the right folding-back ring 23 and folded back is extended rightward after being inserted through the left folding-back ring 33 through which the left inner tightening belt 43 is passed to be folded back, so that the levels of the right folding-back ring 23 and the left folding-back ring 33 are uniformed.

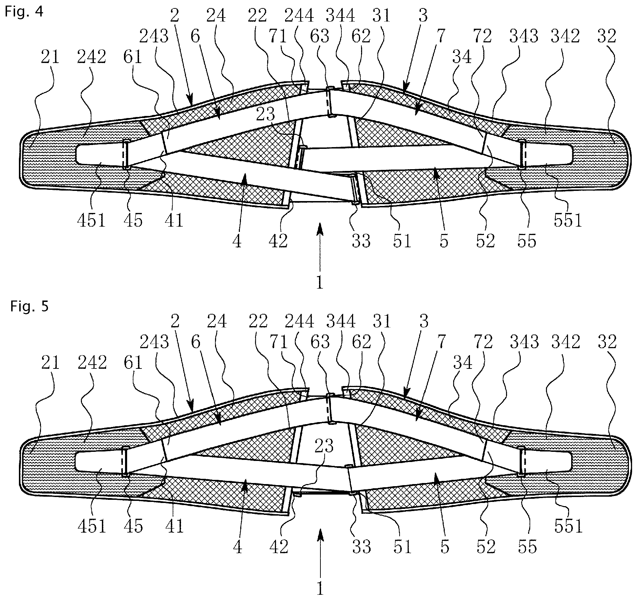

The waist belt 1 of this example is configured to have the left main belt 2 and the right main belt 3 strongly tightened through folding back in two stages, which is achieved on one hand by the left inner tightening belt 43 and left outer tightening belt 44 that are nonstretchable being folded back, and on the other hand by the right inner tightening belt 53 and the right outer tightening belt 54 being tightened likewise. If such strong tightening is not needed, a left tightening belt 4 and a right tightening belt 5 that are nonstretchable can be used while being folded back in one stage, for example, in a manner as in the case of a modification example 2 illustrated in Fig. 4 or a modification example 3 illustrated in Fig. 5. The waist belt 1 of the modification example 2 corresponds to the example (refer to Fig. 1 and Fig. 2) by including the left tightening belt 4 and the right tightening belt 3 are suspended through the left folding-back ring 33 and the right folding-back ring 23, respectively, that are located at upper and lower neighboring positions. The waist belt 1 of the modification example 3 corresponds to the modification example 1 (refer to Fig. 3) with the left folding-back ring 33 and the right folding-back ring 23 the levels of which are uniformed, and with the right tightening belt 3 passed through the left folding-back ring.

In the waist belt 1 of each of the modification example 2 and the modification example 3, the left tightening belt 4 (right tightening belt 5) has the right end 42 (left end 51) thereof connected to the right end 22 (left end 31) of the left main belt 2 (right main belt 3) by utilization of the sewing attachment of the antislip folded-binding part 244 (antislip folded-binding part 344), is passed through the left folding-back ring 33 (right folding-back ring 23) connected to the left end 31 (right end 22) of the right main belt 3 (left main belt 2) and folded back leftward (rightward), and is passed further through the left fixation ring 45 (right fixation ring 55) and folded back rightward (leftward), and has the left end 41 (right end 52) thereof connected to the left end 61 (right end 72) of the left subsidiary tightening belt 6 (right subsidiary tightening belt 7). The left tightening belt 4 (right tightening belt 5) is made of the same nonstretchable belt material as a conventional waist belt or the like of the same kind is made of.

The waist belt 1 of the example is worn on the waist portion 81 of the user 8, for example, in the following manner (while it is obvious that the user 8 is free to follow any manner in which to wear it). In the waist belt 1, the following is performed in advance. The left tab 451 of the left fixation ring 45 and the right tab 551 of the right fixation ring 55 are detached from the female surfaces 242 and 342 of the hook-and-loop fastener that are provided on the outer surface the left main belt 2, so that the right main belt 3, and the left inner tightening belt 43, the left outer tightening belt 44, the right inner tightening belt 53, the right outer tightening belt 54 , and a continuous subsidiary tightening belt 8 are loosened. The user 8 places the thus loosened waist belt 1 on the waist portion 81 of the user 8 in a manner such that the left and right antislip folded-binding parts 244 and 344 can be bilaterally symmetric across the backbone; then winds around the left main belt 2 from the left side to the front side first; then winds around the right main belt 3 from the right side to the front side; and engages the male surface 341 of the hook-and-loop fastener on the left main belt 2 with the female surface 242 of the hook-and-loop fastener. In this manner, the waist belt 1 is wound around the waist portion 81 of the user 8 to the extent that it is kept from falling off.

Starting from this state, the following is performed, so that wearing the waist belt 1 is completed as illustrated in Fig. 6 and Fig. 7. The left tab 451 is pulled from the left side toward the front side; and, at the point where the left inner tightening belt 43, the left outer tightening belt 44, and the left subsidiary tightening belt 6 are adequately tightened, the male surface of the hook-and-loop fastener provided on the left tab 451 is engaged with the female surface 242 provide on the outer surface of the left main belt 2, so that the left fixation ring 45 is fixed. Likewise, the right tab 551 is pulled from the right side toward the front side; and, at the point where the right inner tightening belt 53, the right outer tightening belt 54, and the right subsidiary tightening belt 7 are adequately tightened, the male surface of the hook-and-loop fastener provided on the right tab 551 is engaged with the female surface 342 provide on the outer surface of the right main belt 3, so that the right fixation ring 63 is fixed.

In the waist belt 1 of the example, while the waist belt 1 is worn on the waist portion 81 of the user 8, a set of the left inner tightening belt 43 and the left outer tightening belt 44 and a set of the right inner tightening belt 53 and the right outer tightening belt 54 squeeze tight around the lower half of the waist portion 81, and the left subsidiary tightening belt 6 and the right subsidiary tightening belt 7 squeeze tight around an upper part of the constricted waist portion 81 at the same time, so that the left main belt 2 and the right main belt 3 are brought into wide surface contact with the waist portion 81 (refer to Fig. 7). Here, how much the left inner tightening belt 43, the left outer tightening belt 44, and the left subsidiary tightening belt 6 squeeze and how much the right inner tightening belt 53, the right outer tightening belt 54, and the right subsidiary tightening belt 7 squeeze are made different by how the left tab 451 and the right tab 551 are pulled. Therefore, levels of squeezing in the left and the right sides can be made different while the left main belt 2 and the right main belt 3 are kept making wide surface contact with the waist portion 81.

When the user 8 moves while wearing the waist belt 1, the left subsidiary tightening belt 6 and the right subsidiary tightening belt 7 individually elongate and contract, and the left main belt 2 and the right main belt 3 are kept in tight contact with the upper half of the constricted waist portion 81. At the same time, the elongation and contraction of the left subsidiary tightening belt 6 and the right subsidiary tightening belt 7 absorb the move of the user 8, so that the left outer tightening belt 44 and the right outer tightening belt 54, and, via the left outer tightening belt 44 and the right outer tightening belt 54, the left inner tightening belt 43 and the right inner tightening belt 53 are allowed to follow the move of the user 8 while being pulled and kept tense. Consequently, the left main belt 2 and the right main belt 3 can be kept making wide surface contact with the waist portion 81.

Here, if it is assumed that moves of the left main belt 2 and the right main belt 3 are the same as those of the user 8, the continuous subsidiary tightening belt 8 causes the left subsidiary tightening belt 6 and the right subsidiary tightening belt 7 to elongate and contract by the same lengths as those of the moves of the left main belt 2 and the right main belt 3. The left outer tightening belt 44 and the right outer tightening belt 54 are folded back through the left linking ring 433 and the right linking ring 533, and the moves thereof are therefore halved and transmitted to the left inner tightening belt 43 and the right inner tightening belt 53. In addition, the left inner tightening belt 43 and the right inner tightening belt 53 are folded back through the left folding-back ring 23 and the right folding-back ring 33, and the moves thereof are therefore halved and transmitted to the right end 22 of the left main belt 2 and the left end 31 of the right main belt. Consequently, the left inner tightening belt 43, the left outer tightening belt 44, the right inner tightening belt 53, and the right outer tightening belt 54 in the lower half are kept from being loose, and the continuous subsidiary tightening belt 8 in the upper half stretches by following the moves of the user 8. Thus, the left main belt 2 and the right main belt 3 can be kept making wide surface contact with the waist portion 81.

### Reference Signs List

1 waist belt
2 left main belt
21 left end
22 right end
23 right folding-back ring
24 belt main body
242 female surface of hook-and-loop fastener
243 binding
244 antislip folded-binding part
3 right main belt
31 left end
32 right end
33 left folding-back ring
34 belt main body
341 male surface of hook-and-loop fastener
342 female surface of hook-and-loop fastener
343 binding
344 antislip folded-binding part
4 left tightening belt
41 left end
42 right end
43 left inner tightening belt
431 left end
432 right end
433 left linking ring
44 left outer tightening belt
441 left end
442 right end
45 left fixation ring
451 left tab
5 right tightening belt
51 left end
52 right end
53 right inner tightening belt
531 left end
532 right end
533 right linking ring
54 right outer tightening belt
541 left end
542 right end
55 right fixation ring
551 right tab
6 left subsidiary tightening belt
61 left end
62 right end
63 insertion ring
7 right subsidiary tightening belt
71 left end
72 right end
8 user
81 waist portion

## Claims

1. A waist belt comprising:
a nonstretchable left main belt;
a nonstretchable right main belt;
a nonstretchable left inner tightening belt;
a nonstretchable left outer tightening belt;
a nonstretchable right inner tightening belt;
a nonstretchable right outer tightening belt;
a stretchable left subsidiary tightening belt; and
a stretchable right subsidiary tightening belt,
wherein
the left main belt includes a right folding-back ring in the lower half of the right end thereof,
the right main belt includes a left folding-back ring in the lower half of the left end thereof,
the left inner tightening belt has the right end thereof connected to the lower half of the right end of the left main belt, is passed through the left folding-back ring and folded back leftward, and includes a left linking ring at the left end thereof,
the right inner tightening belt has the left end thereof connected to the lower half of the left end of the right main belt, is passed through the right folding-back ring and folded back rightward, and includes a right linking ring at the right end thereof,
the left subsidiary tightening belt includes an insertion ring configured to have the right subsidiary tightening belt passed therethrough, has the right end thereof joined to the upper half of the left end of the right main belt, and is extended leftward,
the right subsidiary tightening belt has the left end thereof joined to the upper half of the right end of the left main belt and is passed through the insertion ring and extended rightward,
the left outer tightening belt has the right end thereof connected to the left end of the left subsidiary tightening belt, is passed through a left fixation ring and folded back rightward, is passed through the left linking ring and folded back leftward, and has the left end thereof connected to the left main belt, the left fixation ring being attachable to and detachable from the outer surface of the left main belt, and
the right outer tightening belt has the left end thereof connected to the right end of the right subsidiary tightening belt, is passed through a right fixation ring and folded back leftward, is passed through the right linking ring and folded back rightward, and has the right end thereof connected to the right main belt, the right fixation ring being attachable to and detachable from the outer surface of the right main belt.

2. A waist belt comprising:
a nonstretchable left main belt;
a nonstretchable right main belt;
a nonstretchable left tightening belt;
a nonstretchable right tightening belt;
a stretchable left subsidiary tightening belt; and
a stretchable right subsidiary tightening belt,
wherein
the left main belt includes a right folding-back ring in the lower half of the right end thereof,
the right main belt includes a left folding-back ring in the lower half of the left end thereof,
the left subsidiary tightening belt includes an insertion ring configured to have the right subsidiary tightening belt passed therethrough, has the right end thereof joined to the upper half of the left end of the right main belt, and is extended leftward,
the right subsidiary tightening belt has the left end thereof joined to the upper half of the right end of the left main belt, and is passed through the insertion ring and extended rightward,
the left tightening belt has the right end thereof connected to the lower half of the right end of the left main belt, is passed through the left folding-back ring and folded back leftward, is passed through the left fixation ring and folded back rightward, and has the left end thereof connected to the right end of the left subsidiary tightening belt, the left fixation ring being attachable to and detachable from the outer surface of the left main belt, and
the right tightening belt has the left end thereof connected to the lower half of the left end of the right main belt, is passed through the right folding-back ring and folded back rightward, is passed through a right fixation ring and folded back leftward, and has the [[left]] J right end thereof connected to the right end of the right subsidiary tightening belt, the right fixation ring being attachable to and detachable from the outer surface of the right main belt.

3. The waist belt according to claim 1 or 2, wherein
the insertion ring is provided to the right subsidiary tightening belt instead of the left subsidiary tightening belt,
the left subsidiary tightening belt has the right end thereof joined to the upper half of the left end of the right main belt, and is passed through the insertion ring and extended leftward, and
the right subsidiary tightening belt has the left end thereof joined to the upper half of the right end of the left main belt and is extended rightward.

4. The waist belt according to any one of claims 1 to 3, wherein the left main belt and the right main belt are integrally wound around the waist portion of the user with one part of a hook-and-loop fastener and the other part of the hook-and-loop fastener formed so as to be attachable to and detachable from each other, the one part being provided on either or both of the inner surfaces of the left end of the left main belt and the right end of the right main belt, the other part being provided on either or both of the outer surfaces of the left main belt and the right main belt.

5. The waist belt according to any one of claims 1 to 4, wherein, while the left fixation ring is positionally fixed with respect to an outer surface of the left main belt with a part of a hook-and-loop fastener and the other part thereof formed so as to be attachable to and detachable from each other, the one part being provided to the left fixation ring, the other part being provided on the outer surface of the left main belt, the right fixation ring is positionally fixed with respect to an outer surface of the right main belt with one part of a hook-and-loop fastener and the other part thereof formed so as to be attachable to and detachable from each other, the one part being provided to the right fixation ring, the other part being provided on the outer surface of the right main belt.
